# EUROPÄISCHE PATENTSCHRIFT

(11) **EP 0 709 072 B1**
(45) Veröffentlichungstag und Bekanntmachung des Hinweises auf die Patenterteilung: **02.05.2002**
(21) Anmeldenummer: 95115227.1
(22) Anmeldetag: 29.11.1991
(51) Int. Cl.: A61F 2/38, A61B 17/14, A61B 17/16

(54) **Vorrichtung zur Plazierung einer Bohrung in einem Knochen**
Device for positioning a hole in a bone
Dispositif pour positionner un trou dans un os

(30) Priorität: 29.11.1990 DE 4038037
(43) Veröffentlichungstag der Anmeldung: 01.05.1996
(62) Teilanmeldung aus: 91120536.7
(73) Patentinhaber: GMT Gesellschaft für medizinische Technik mbH, 22767 Hamburg (DE); Waldemar Link (GmbH & Co.), 22339 Hamburg (DE)
(72) Erfinder: Engelbrecht Edgar Dr., 22301 Hamburg (DE); Nieder Elmar Dr., 21635 Jork (DE); Keller Arnold, 23863 Kayhude (DE)
(74) Vertreter: Glawe, Delfs, Moll & Partner

(56) Entgegenhaltungen:
- EP-A- 0 153 831
- US-A- 3 782 373
- US-A- 4 257 411

## Beschreibung

Die Erfindung betrifft eine Vorrichtung zur Plazierung einer Bohrung in einem Knochen mit einer zentrisch zur einzubringenden Bohrung ausrichtbaren Justierung, die mittels eines Abstandshalters mit einem Abstand zu einer Bohrhülse angeordnet ist, in welcher ein die Bohrung herstellender Bohrer führbar ist. Eine solche Vorrichtung ist aus der EP-A-0 153 831 bekannt.

Scharniergelenkendoprothesen sind als Kniegelenkendoprothesen in der Fachwelt bekannt und werden erfolgreich insbesondere dann implantiert, wenn sich herausstellt, daß bei einem zu behandelnden Patienten der das Kniegelenk umgebende Weichteilapparat nicht mehr stark genug ausgebildet ist, um den Femurteil mit der notwendigen Kraft im Wirkzusammenhang mit dem Tibiateil zu halten.

Bei der Implantation von Scharniergelenkendoprothesen mit nachträglich einsetzbaren Lagerzapfen ist es erforderlich, die am Knochen vorzunehmende Bohrung mit hoher Präzision durchzuführen, um eine zusätzliche Schwächung der Knochensubstanz zu vermeiden.

Aufgabe der vorliegenden Erfindung ist es daher, eine Vorrichtung anzugeben, mit deren Hilfe die erforderliche Bohrung mit hoher Präzision in den Knochen eingebracht werden kann.

Diese Aufgabe wird erfindungsgemäß dadurch gelöst, daß quer zur Bohrachse der Bohrhülse ein Stiel am Abstandshalter angeordnet ist, entlang dem ein Abstützelement auf einer Führungshülse geführt ist, die über einen Tragarm mit einer Stütze verbunden ist, die von einem Innenraum eines Gehäuses aufnehmbar ist, welches einen Lagerzapfen führt, um den ein Tibiateil drehbar bezüglich eines Femurteils einer Kniegelenkendoprothese gelagert ist.

Die erfindungsgemäße Ausbildung der Vorrichtung ermöglicht es dem operierenden Chirurgen innerhalb kurzer Zeit und mit hoher Präzision, die Bohrung in den Knochen einzubringen. Durch die Benutzung der Vorrichtung wird der Chirurg von manuellen Ausrichtungen und gegebenenfalls erforderlichen Richtungskorrekturen entlastet. Die Justierung kann in eine den einzubringenden Lagerzapfen aufnehmende Lagerschale eingebracht werden. Hierdurch wird eine exakte zentrische Ausrichtung der Bohrung in Relation zum einzubringenden Lagerzapfen erreicht.

Gemäß einer bevorzugten Ausführungsform der Erfindung ist ein entlang des Stieles der Vorrichtung verschiebliches Abstützelement vorgesehen. Dieses Abstützelement kann nach einem Einbringen der Justierung in den Bereich der Lagerschale in den Gehäuseinnenraum des Tibiateils eingebracht und dort bezüglich des Stieles fixiert werden und verhindert zuverlässig ein Verkanten der Vorrichtung. Das Abstützelement füllt während des Bohrvorganges mit einer Stütze einen sich zwischen den Lagerschalen erstreckenden freien Zwischenraum aus. Durch das Zusammenwirken der Justierung und des Abstützelementes besitzt die Kombination aus Vorrichtung und Femurteil während des Bohrvorganges keine Bewegungsfreiheit in der seitlichen Verkantung, sondern nur in der Beugeebene. Relativbewegungen der Teile zueinander quer zur Beugeebene können somit ausgeschlossen werden. Auch bei während des Bohrvorganges einwirkenden Kräften ist somit für eine genaue Plazierung und exakte Ausführung der Bohrung gesorgt, bei deren Herstellung ein Hohlbohrer verwendet wird. Mit seiner Hilfe wird in der Verlängerung der Schwenkachse ein Knochenpfropf aus dem den Femurteil umgebenden Knochen entfernt. Durch das dadurch im Knochen entstehende Loch wird ein Lagerzapfen zur Verbindung des Femurteils mit dem Tibiateil in das Kniegelenk eingeführt. Nachdem dieser Lagerzapfen montiert worden ist, wird der Knochenpfropf wieder in das ausgebohrte Loch des Knochens eingeseetzt, wo er eine natürliche Verbindung mit diesem herstellt und ihn versteift.

Weitere Einzelheiten und Merkmale der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

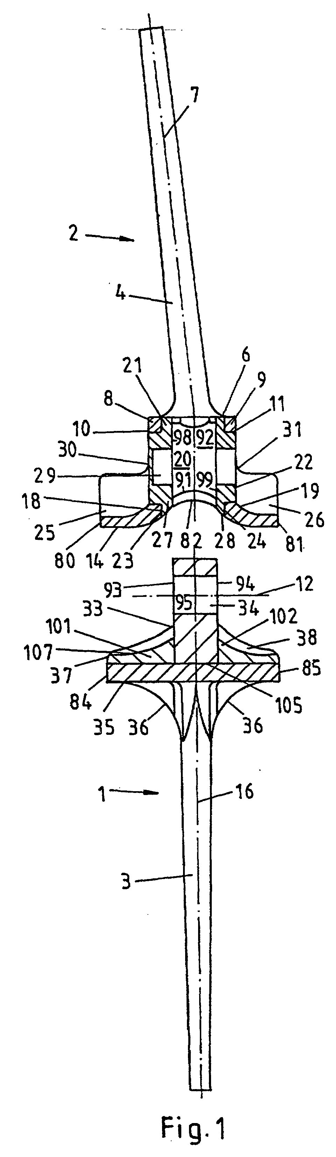

- Fig. 1:: Einen Längsschnitt durch eine Endoprothese mit vom Tibiateil abgenommenen Femurteil.

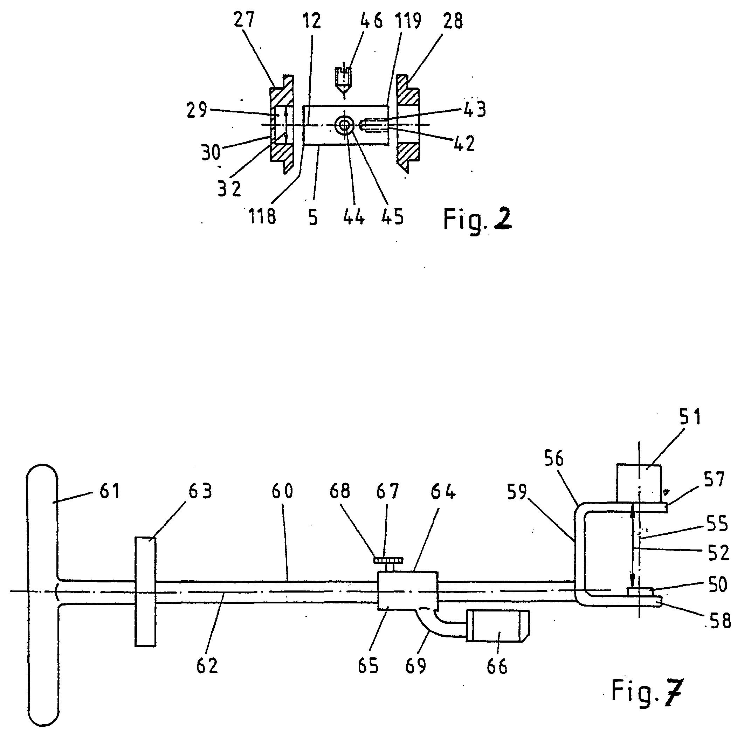

- Fig. 2:: einen Längsschnitt durch zwei zur Aufnahme des Lagerzapfens vorgesehene Lagerschalen und eine Ansicht eines Lagerzapfens mit einer Zentrierschraube,

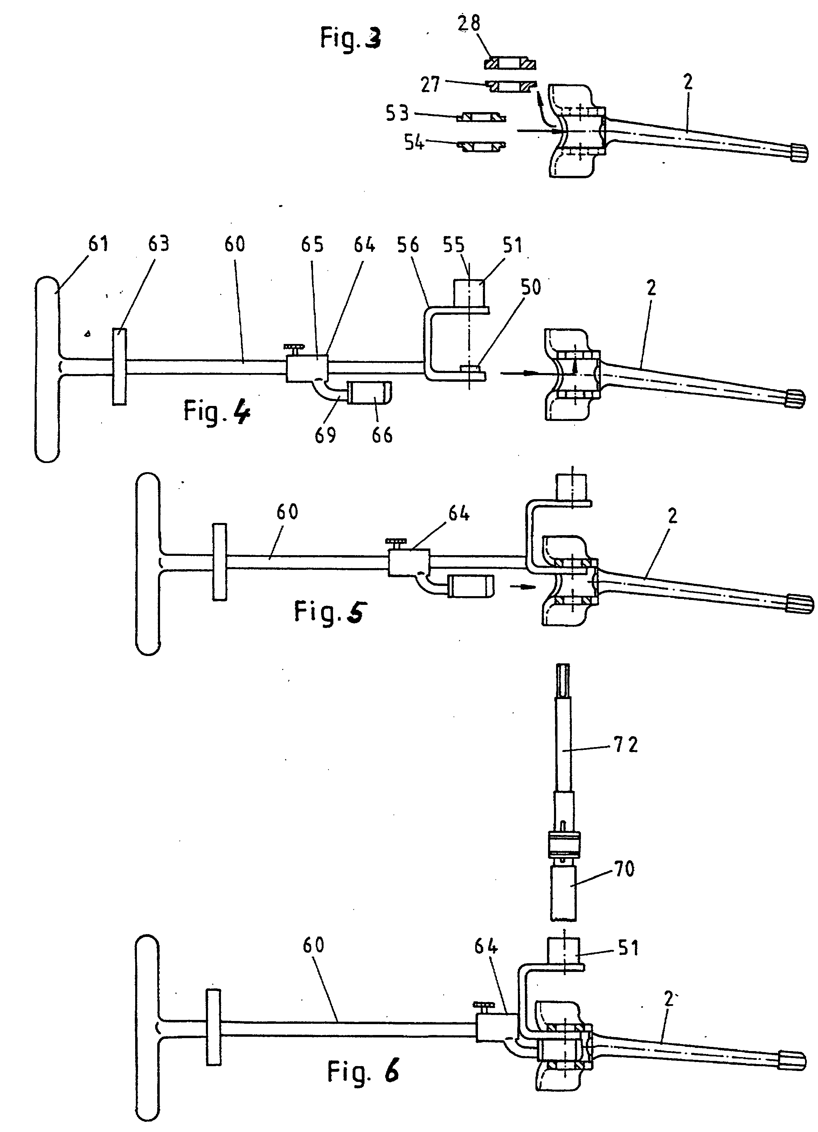

- Fig. 3:: eine schematische Darstellung der Vorbereitung des Femurteils zur Aufnahme der Vorrichtung zur Plazierung der Bohrung,
- Fig. 4:: eine schematische Darstellung der relativen Anordnung der Vorrichtung zum Femurteil vor dem Einsetzen der Vorrichtung,
- Fig. 5:: eine schematische Darstellung einer in das Femurteil eingesetzten Vorrichtung,
- Fig. 6:: eine schematische Darstellung der relativen Anordnung eines Bohrers zur Vorrichtung vor Beginn des Bohrvorganges,
- Fig. 7:: eine Seitenansicht einer Vorrichtung zur Plazierung einer Bohrung,

Eine Kniegelenkendoprothese besteht im wesentlichen aus einem Tibiateil (1) und einem Femurteil (2). Das Tibiateil (1) weist einen Tibiaschaft (3), das Femurteil (2) einen Femurschaft (4) auf. Die beiden Schäfte (3, 4) weisen in einander abgewandte Richtungen und dienen zur Befestigung der Teile (1, 2) in einem ihnen jeweils benachbarten Knochen.

Im Bereich der dem Tibiateil (1) zugewandten Ausdehnung des Femurteils (2) ist ein einen Lagerzapfen (5) führendes Gehäuse (6) angeordnet. Dieser Lagerzapfen ist im Tibiateil (1) befestigt und im Femurteil (2) drehbar gelagert. Bei der Ausführung von Drehbewegungen des Lagerzapfens (5) führen die beiden Teile (1, 2) Schwenkbewegungen in einer Beugeebene gegeneinander aus. Diese Beugebewegungen führen von einer gestreckten-Stellung, in der sich durch jeden der beiden Schäfte (3, 4) eine senkrecht zur Beugeebene verlaufende Schaftebene erstreckt, in eine verschwenkte Stellung, in der eine sich durch den Femurschaft (4) erstreckende Femurlängsachse (7) mit einer sich durch den Tibiaschaft (3) erstreckenden Tibialängsachse (16) einen Beugewinkel zwischen sich einschließt. Dieser Beugewinkel umschließt einen hinteren Teil des Femurteils (2) und einen hinteren Teil des Tibiateils (1).

Im Bereich seiner sich im wesentlichen planparallel zur Beugeebene erstreckenden seitlichen Begrenzungen (8, 9) weist das Gehäuse (6) Ausnehmungen (10, 11) auf.

Die Ausnehmungen (10, 11) dienen zur Aufnahme von Lagerschalen (27, 28), in denen der Lagerzapfen (5) gelagert ist. Diese Lagerschalen (27, 28) besitzen jeweils einen Bund (23, 24), mit dem sie in die Ausnehmungen (10, 11) hineinragen. An diesen Bund (23, 24) schließen sich Auflageflächen (21, 22) an, mit denen die Lagerschalen (27, 28) in einen vom Gehäuse (6) umschlossenen Gehäuseinnenraum (20) hineinragen.

Eine Vorrichtung zur Plazierung einer Bohrung in einem Knochen weist im wesentlichen eine Justierung (50) sowie eine Bohrhülse (51) auf, die in einem Abstand (52) zueinander angeordnet sind. Die Justierung (50) ist im wesentlichen zylindrisch ausgebildet und dem Innendurchmesser der Lagerschalen (27, 28) angepaßt. Es ist aber auch möglich, während der Einbringung der Bohrung in den Knochen statt der Lagerschalen (27, 28) Arbeitslagerschalen (53, 54) zu verwenden. Die Justierung (50) sowie die Bohrhülse (51) sind zentrisch zu einer Bohrachse (55) angeordnet. Im Bereich ihrer der Justierung (50) zugewandten Begrenzung mündet die Bohrhülse (51) in einen Abstandshalter (56) ein. Der Abstandshalter (56) ist im wesentlichen U-förmig ausgebildet und trägt im Bereich seines einen Seitenschenkels (57) die Bohrhülse (51). Im Bereich der der Bohrhülse (51) zugewandten Begrenzung des anderen Seitenschenkels (58) ist die Justierung (50) angeordnet. Die Seitenschenkel (57, 58) sind durch einen Basisschenkel (59) miteinander verbunden. Im Bereich seiner den Seitenschenkeln (57, 58) abgewandten Begrenzung mündet der Basisschenkel (59) in einen Stiel (60), der sich im wesentlichen parallel zur Richtung der Seitenschenkel (57, 58) erstreckt. Im Bereich seiner den Seitenschenkeln (57, 58) abgewandten Ausdehnung mündet der Stiel (60) in einen Griff (61), der sich im wesentlichen quer zu einer Stiellängsachse (62) erstreckt.

Auf dem Stiel (60) ist im Bereich zwischen dem Basisschenkel (59) und dem Griff (61) entlang der Stiellängsachse (62) verschieblich ein Anschlagring (63) gleitend gelagert, der mit seiner dem Griff (61) abgewandten Stirnfläche einem Abstützelement (64) benachbart ist und dieses beim Verschieben entlang der Stiellängsachse (62) beaufschlagt. Dieses Abstützelement (64) ist entlang der Stiellängsachse (62) verschieblich angeordnet und weist eine den Stiel (60) bereichsweise umschließende Führungshülse (65) sowie eine Stütze (66) auf. Die Führungshülse (65) ist mit Hilfe einer Arretierung (67), die beispielsweise als Schraube (68) ausgebildet ist, in ihrer Position relativ zum Stiel (60) fixierbar. Die Führungshülse (65) ist über einen Tragarm (69) mit der Stütze (66) verbunden.

Die Stütze (66) weist eine an den Gehäuseinnenraum (20) angepaßte Ausbildung auf. In Richtung der Schwenkachse (12) weist die Stütze (66) eine Bemaßung auf, die zusammen mit der Bemaßung des Seitenschenkels (58) der Innenbemaßung des Gehäuseinnenraumes (20) in Richtung der Schwenkachse (12) entspricht. Die Bohrhülse (51) ist zur Aufnahme eines Topffräsers (70) vorgesehen. Der Topffräser (70) ist über eine Arretierung (71) auswechselbar mit einem Bohrerschaft (72) verbunden.

Zur Implantation der Kniegelenkendoprothese wird zunächst der Tibia- und der Femurknochen zur Aufnahme der Schäfte (3, 4) vorbereitet. Nach dem Einsetzen der Schäfte (3, 4) und deren Fixierung relativ zu dem sie aufnehmenden Knochen wird in den Gehäuseinnenraum (20) der Seitenschenkel (58) eingeführt und die Justierung (50) im Bereich der Lagerschale (30), bzw. im Bereich einer der Arbeitslagerschalen (53, 54) positioniert. Anschließend wird das Abstützelement (64) in Richtung auf den Gehäuseinnenraum (20) verschoben und mit der Stütze (66) in den Gehäuseinnenraum (20) eingeführt. Sollten dabei Schwierigkeiten auftreten, beispielsweise das Abstützelement (64) nicht ohne Schwierigkeit in das Gehäuse (6) hineingleiten oder im Gehäuse (6) nicht gut ausgerichtet sein, so kann seine Lage durch vorsichtige Schläge mit dem Anschlagring (63) korrigiert werden, bis die Bohrhülse (51) genau auf das herzustellende Bohrloch ausgerichtet ist. Nach einer Festlegung des Abstützelementes (64) in Relation zum Stiel (60) durch Festziehen der Arretierung (67) ist die Vorrichtung spielfrei im Bereich des Gehäuses (6) gehaltert. Die Bohrachse (55) fällt nun mit der Schwenkachse (12) zusammen. Mit Hilfe des Topffräsers (70), der durch die Bohrhülse (51) in Richtung auf den Femurknochen geführt wird, kann ein Knochenpfropf aus den Femurknochen entfernt werden. Nach dem Einsetzen des Lagerzapfens (5), der zu diesem Zwecke mit seinem Innengewinde (43) auf ein Außengewinde (73) eines Montagewerkzeuges (74) aufgeschraubt wird, wird der Lagerzapfen (5) mit Hilfe der Madenschraube (46), die durch die Bohrung (41) dem Innengewinde (45) zugeführt wird, in Relation zum Femurteil (2) arretiert. Zur Wiederherstellung der Knochenfestigkeit wird der mit Hilfe des Topffräsers (70) entfernte Knochenpfropf wieder in die im Femurknochen erzeugte Ausnehmung eingesetzt. Vor dem Einführen des Lagerzapfens (5) werden das Tibiateil (1) und das Femurteil (2) relativ so zueinander positioniert, daß die Ausnehmungen (10, 11) des Femurteils und die Ausnehmung (34) des Tibiateils zentrisch zu einer gemeinsamen Schwenkachse (12) ausgerichtet sind. Nunmehr wird durch die Ausnehmungen (10, 11, 34) eine leicht montierbare und anschließend wieder demontierbare Probeachse hindurchgeführt, mit deren Hilfe ausprobiert wird, ob alle Teile richtig montiert und gut gegeneinander beweglich sind. Gegebenenfalls können Korrekturen vorgenommen werden. Wenn alle Teile richtig montiert und ausgerichtet sind, wird die Probeachse entfernt und der Lagerzapfen (5) eingepaßt, der dafür sorgt, daß die Gelenkteile in der gewünschten Art zusammenwirken. Die Montage des Lagerzapfens (5) erfolgt in der Weise, daß die Madenschraube (46) mit einem Schraubendreher (75) festgezogen wird. Die bei der Implantation der Endoprothese manuell einzubringenden Hebelkräfte können mit Hilfe eines Hebelwerkzeuges (76) erzeugt werden.

## Patentansprüche

1. Vorrichtung zur Plazierung einer Bohrung in einem Knochen mit einer zentrisch zur einzubringenden Bohrung ausrichtbaren Justierung (50), die mittels eines Abstandshalters (56) mit einem Abstand (52) zu einer Bohrhülse (51) angeordnet ist, in welcher ein die Bohrung herstellender Bohrer (17) führbar ist, **dadurch gekennzeichnet, daß** quer zur Bohrachse (55) der Bohrhülse (51) ein Stiel (60) am Abstandshalter (56) angeordnet ist, entlang dem ein Abstützelement (64) auf einer Führungshülse (65) geführt ist, die über einen Tragarm (69) mit einer Stütze (66) verbunden ist, die von einem Innenraum (20) eines Gehäuses (6) aufnehmbar ist, welches einen Lagerzapfen (5) führt, um den ein Tibiateil (1) drehbar bezüglich eines Femurteils (2) einer Kniegelenkendoprothese gelagert ist.

2. Vorrichtung nach Anspruch 1, **dadurch gekennzeichnet, daß** der Abstandshalter (56) im wesentlichen U-förmig ausgebildet ist und einen die Bohrhülse (51) halternden Seitenschenkel (57) aufweist sowie einen die Justierung (50) halternden Seitenschenkel (58) aufweist.

3. Vorrichtung nach einem der Ansprüche 1 oder 2, **dadurch gekennzeichnet, daß** der Abstandshalter (56) einen Basisschenkel (59) aufweist, der im Bereich seiner den Seitenschenkeln (57, 58) abgewandten Begrenzung in den Stiel (60) einmündet.

4. Vorrichtung nach einem der Ansprüche 1 bis 3, **dadurch gekennzeichnet, daß** der Stiel (60) im Bereich seiner dem Basisschenkel (59) abgewandten Begrenzung in einen Griff (61) einmündet.

5. Vorrichtung nach Anspruch 4 **dadurch gekennzeichnet, daß** sich der Griff (61) im wesentlichen quer zu einer Stiellängsachse (62) erstreckt.

6. Vorrichtung nach einem der Ansprüche 1 bis 5, **dadurch gekennzeichnet, daß** im Bereich der Führungshülse (65) eine das Abstützelement (64) lösbar gegenüber dem Stiel (60) fixierende Arretierung (67) vorgesehen ist.

7. Vorrichtung nach Anspruch 6, **dadurch gekennzeichnet, daß** die Arretierung (67) als Schraube (68) ausgebildet ist.

8. Vorrichtung nach einem der Ansprüche 1 bis 7, **dadurch gekennzeichnet, daß** die Stütze (66) in Richtung einer Bohrachse (55) eine Bemaßung aufweist, die zusammen mit der Bemaßung des Seitenschenkels (57) der Bemaßung des Gehäuseinnenraumes (20) entlang der Bohrachse (55) entspricht.

9. Vorrichtung nach einem der Ansprüche 1 bis 8, **dadurch gekennzeichnet, daß** die Justierung (50) in Relation zur Bohrachse (55) zentrisch im Bereich einer den Lagerzapfen (5) aufnehmenden Lagerschale (28) positionierbar ist.

## Claims

1. An apparatus for positioning a bore in a bone with an adjustment means (50) which can be aligned centrally to the bore to be introduced and which is arranged by means of a spacer (56) at a distance (52) from a bore sleeve (51), in which a drill (17) forming the bore can be guided, **characterised in that** transversely to the bore axis (55) of the bore sleeve (51) a shaft (60) is arranged on the spacer (56), along which a support element (64) is guided on a guide sleeve (65) which is connected via support arm (69) to a support (66) which can be accommodated by an inner space (20) of a housing (6) which guides a journal pin (5), about which a tibia part (1) is rotatable relative to a femur part (2) of knee joint endoprosthesis.

2. An apparatus according to Claim 1, **characterised in that** the spacer (56) is substantially U-shaped and has a side arm (57) retaining the bore sleeve (51), as well as a side arm (58) retaining the adjustment means (50).

3. An apparatus according to Claim 1 or 2, **characterised in that** the spacer (56) has a base arm (59) which in the vicinity of its boundary remote from the side arms (57,58) merges into the shaft (60).

4. An apparatus according to any one of Claims 1 to 3, **characterised in that** in the vicinity of its boundary remote from the base arm (59) the shaft (60) merges into a handle (61).

5. An apparatus according to Claim 4, **characterised in that** the handle (61) extends substantially transversely to the longitudinal axis (62) of the shaft.

6. An apparatus according to any one of Claims 1 to 5, **characterised in that** in the vicinity of the guide sleeve (65) a stop means (67) is provided which releasably locates the support element (64) with respect to the shaft (60).

7. An apparatus according to Claim 6, **characterised in that** the stop means (67) is in the form of a screw (68).

8. An apparatus according to any one of Claims 1 to 7, **characterised in that** in the direction of a bore axis (55) the support (66) has dimensions which together with the dimensions of the side arm (57) correspond to the dimensions of the housing inner space (20) along the bore axis (55).

9. An apparatus according to any one of Claims 1 to 8, **characterised in that** the adjustment means (50) can be positioned in relation to the bore axis (55) centrally in the vicinity of a bearing shell (28) accommodating the journal pin (5).

## Revendications

1. Dispositif pour positionner un trou dans un os, comportant un dispositif d'ajustage (50) qui peut être orienté centré par rapport au trou à pratiquer et qui est disposé, au moyen d'un écarteur (56), à une distance (52) d'une douille de perçage (51) dans laquelle peut être guidé un foret (17) réalisant le trou, **caractérisé en ce que** transversalement à l'axe de perçage (55) de la douille de perçage (51) est disposée, sur l'écarteur (56), une tige (60) le long de laquelle un élément d'appui (64) est guidé sur un manchon de guidage (65) lequel est relié, par un bras de support (69), à un appui (66) qui peut être reçu par un volume intérieur (20) d'un boîtier (6), lequel guide un tourillon (5) autour duquel un tibia (1) est monté de manière à pouvoir tourner autour d'un fémur (2) d'une endoprothèse d'articulation du genou.

2. Dispositif selon la revendication 1, **caractérisé en ce que** l'écarteur (6) est sensiblement en forme de U et comporte une branche latérale (57), maintenant la douille de perçage (51), ainsi qu'une branche latérale (58) qui maintient le dispositif d'ajustage (50).

3. Dispositif selon l'une des revendications 1 ou 2, **caractérisé en ce que** l'écarteur (56) comporte une branche de base (59) qui débouche dans la tige (60), dans la zone de sa délimitation tournée à l'opposé des branches latérales (57, 58).

4. Dispositif selon l'une des revendications 1 à 3, **caractérisé en ce que** la tige (6) débouche dans une poignée (61), dans la zone de sa délimitation tournée à l'opposé de la branche de base (59).

5. Dispositif selon la revendication 4, **caractérisé en ce que** la poignée (61) s'étend sensiblement transversalement à un axe longitudinal (62) de la tige.

6. Dispositif selon l'une des revendications 1 à 5, **caractérisé en ce qu'**il est prévu, dans la zone de la douille de guidage (65), un dispositif d'arrêt (67) qui fixe l'élément d'appui (64) de manière amovible par rapport à la tige (60).

7. Dispositif selon la revendication 6, **caractérisé en ce que** le dispositif d'arrêt (67) est réalisé en tant que vis (68).

8. Dispositif selon l'une des revendications 1 à 7, **caractérisé en ce que** l'appui (66) présente, en direction d'un axe de perçage (55), une dimension qui, avec la dimension du côté latéral (57), correspond à la dimension du volume intérieur (20) du boîtier le long de l'axe de perçage (55).

9. Dispositif selon l'une des revendications 1 à 8, **caractérisé en ce que** le dispositif d'ajustage (50) peut être positionné par rapport à l'axe de perçage (55), de manière centrée dans la zone d'une coquille de coussinet (28) recevant le tourillon (5).
